# EUROPÄISCHE PATENTSCHRIFT

(11) **EP 0 667 333 B1**
(45) Veröffentlichungstag und Bekanntmachung des Hinweises auf die Patenterteilung: **05.11.1997**
(21) Anmeldenummer: 95100664.2
(22) Anmeldetag: 19.01.1995
(51) Int. Cl.: C07C 51/47, C07C 59/265, C07C 59/08

(54) **Verfahren zur Abtrennung von Hydroximono- und Tricarbonsäuren**
Process for separating hydroxy mono- and tricarboxylic acids
Procédé de séparation d'acides hydroxy mono- et tricarboxyliques

(30) Priorität: 09.02.1994 DE 4403987
(43) Veröffentlichungstag der Anmeldung: 16.08.1995
(73) Patentinhaber: Degussa Aktiengesellschaft, 60387 Frankfurt am Main (DE)
(72) Erfinder: Sextl, Elfriede, Dr., D-63826 Geiselbach (DE); Kiss, Akos, Dr., D-63457 Hanau (DE); Kinz, Heike, D-63457 Hanau (DE); Schäfer-Treffenfeldt, Wiltrud, Dr., D-63179 Obertshausen (DE); Yonsel, Sems, Dr., TR-80860-Istinye-Istanbul (TR); Stockhammer, Stefan, D-63579 Freigericht-Neuses (DE)

(56) Entgegenhaltungen:
- EP-A- 0 132 049
- EP-A- 0 442 181
- US-A- 2 253 061
- US-A- 2 882 244

## Beschreibung

Die Erfindung betrifft die Abtrennung von Hydroxymono- und tricarbonsäuren aus wäßrigen Lösungen durch Adsorption an Zeolithen.

Wichtige Vertreter dieser Säuren sind die Citronen- und die Milchsäure, die in großen Mengen überwiegend auf dem Weg der Fermentation gewonnen werden.

Als wirksamstes Reinigungsverfahren hat sich für die Citronensäure die Fällung des relativ schwerlöslichen Calciumcitrats durchgesetzt. Aus diesem Salz wird die reine Säure durch Zusatz von Schwefelsäure und Ausfällen von Gips gewonnen. So entstehen mit jeder Tonne Citronensäure rd. 2,5 t Nebenprodukte, deren größten Anteil mit 65 % Gips bildet.

Die komplexbildenden Eigenschaften der Citronensäure bewirken, daß die Löslichkeit von Gips und anderen organischen Salzen in den Betriebslösungen rund 10mal so hoch ist wie in Wasser. Die unmittelbare Eindampfung dieser Lösung führt deshalb zu starken Verkrustungen von Heizkörpern und Rohrleitungssystemen durch Abscheidung von Gips. Alle modernen Verfahren arbeiten daher mindestens mit einer Kationenaustauschstufe, in der Calcium- und andere Kationen durch Wasserstoffionen ersetzt werden. Die ebenfalls in der Lösung vorhandenen Sulfationen können entweder mit Bariumcarbonat gefällt oder über Anionenaustauscher entfernt werden. (Ullmanns Enzyklopädie, Bd. 9, 4. Auflage 1975, 632)

Die Abtrennung der Milchsäure aus den Fermentationsbrühen und die dazugehörige Aufreinigung verläuft ebenfalls in den meisten Fällen über Anionen- und Kationenaustauscher (EP-A 0 517 242, EP-A 0 393 818)

Aus der EP-A 0 442 181 ist ein Prepuls-Verfahren bekannt, um gesättigte Hydroxidicarbonsäuren von ungesättigten Dicarbonsäuren zu trennen. Letztere werden dabei an einem nicht-zeolithischen Siliciumdioxid-Molekularsieb (Silicalit) stärker adsorbiert und damit von der gesättigten Säure, in diesem Fall Äpfelsäure, abgetrennt. Die Desorption erfolgt mit einem organischen Lösungsmittel/Wassergemisch.

Gemäß der in dieser Anmeldung verwendeten Definition besitzt der Silicalit den Modul ∞, da er Al₂O₃-frei ist.

Die Aufgabe der Erfindung besteht nun darin, ein einfaches Verfahren der Abtrennung zur Verfügung zu stellen.

Gegenstand der Erfindung ist ein Verfahren zur Abtrennung von gesättigten Hydroximono- und tricarbonsäuren aus wäßrigen Lösungen durch Adsorption, das dadurch gekennzeichnet ist, daß man eine eine dieser Carbonsäuren enthaltende wäßrige Lösung unter Adsorptionsbedingungen mit einem adsorbierenden Material enthaltend einen Zeolith, bevorzugt mit einem Modul (SiO₂/Al₂O₃-Verhältnis) bis 400, in Kontakt bringt und die entsprechende Carbonsäure durch Desorption daraus gewinnt. Die Ausgangslösungen stammen bevorzugt aus den bei der Herstellung dieser Säuren anfallenden Fermentationsbrühen.

Bevorzugt setzt man dieses Verfahren zur Isolierung von Citronensäure und Milchsäure bzw. deren Alkalisalzen ein.

Unter Adsorptionsbedingungen versteht man einen pH-Bereich von 0,5 bis 5, bevorzugt 1 bis 4,0, wobei die Temperaturen im allgemeinen innerhalb eines Bereichs von 15 bis 50° C, bevorzugt 20 bis 30° C, liegen.

Der Zeolith kann sowohl in Pulverform in einer Suspension als auch in granulierter Form oder als Formkörper (z.B. Zylinder) in einem Festbett eingesetzt werden.

Als besonders geeignet für die Adsorption von Citronensäure hat sich ein dealuminierter Y-Zeolith (DAY) mit einem Modul im Bereich von 20 bis 400, bevorzugt bis 250 erwiesen. Der Porendurchmesser des dealuminierten Y-Zeoliths beträgt ca. 0,8 nm. Zeolithe dieses Typs sind an sich bekannt (Kirk-Othmer, The Enzyclopedia of Chemical Technology, Third Ed., Vol. 15, 638 - 669).

Milchsäure wird durch diesen Zeolithtyp ebenso adsorbiert, aber auch insbesondere durch Zeolithe vom Typ ZSM-5 oder Mordenit mit einem Modul zwischen 15 und 400. Alle Zeolithe werden in diesem Verfahren bevorzugt in der H- oder Na-Form eingesetzt.

Die Desorption der adsorbierten Säuren erfolgt, indem man den abgetrennten Festkörper mit einer Alkalihydroxid enthaltenden oder ammoniakalischen Lösung behandelt. Im letzteren Fall wählt man eine NH3-Konzentration von 0,1 bis 3 g NH₃/lH₂O und setzt diese Lösung in einem Gewichts-Verhältnis von bis zu 10 : 1, bezogen auf die Menge des Zeoliths, ein. Die Desorptionslösung wird bevorzugt mehrfach z. B. über eine Säule, die mit dem Zeolith gefüllt ist, gefahren. Man erhält so die entsprechenden Salze der Säuren.

Der pH-Wert sinkt mit fortschreitender Freisetzung der adsorbierten Säuren ab, sollte jedoch nicht den Wert 5 unterschreiten, um eine erneute Adsorption zu vermeiden.

Es ist auch möglich, unter Berücksichtigung dieses Umstands mit neutralem Wasser zu desorbieren. Man erhält dann die reinen Säuren.

In der Festbettsäule durchgeführte Adsorptions-Desorptions-versuche mit Citronensäure an DAY Modul 200 zeigen, daß über eine Zyklenzahl von 10 Adsorptions- und Desorptions-kapazität im Rahmen des Meßfehlers konstant bleiben.

Ein Vorteil des erfindungsgemäßen Verfahrens liegt auch darin, daß als Ausgangslösungen die bei der fermentativen Herstellung der Säuren anfallenden Lösungen eingesetzt werden können, bevorzugt nach der Abtrennung von gegebenenfalls vorliegenden Feststoffen, insbesondere aber der Biomasse.
Bekannterweise zeigt sich in Abhängigkeit von der C-Quelle ein bestimmtes Spektrum an häufig nicht näher spezifizierten Nebenprodukten und Verunreinigungen.
Es hat sich erwiesen, daß in Fermentationsbrühen auf der Basis von Melasse mit Mordenit eine gute Trennleistung erreicht werden kann, während Zeolithe des Typs DAY, insbesondere DAY 200 eine gute Abtrennung aus Glucosemedium ermöglichen (s. Tab. 1).
Nebenprodukte wie Oxalsäure, Fumarsäure oder Essigsäure kann man gezielt durch eine Behandlung der Lösungen mit einem Zeolith des Typs ZSM-5 abtrennen, an die sich die Adsorption der gewünschten herzustellenden Säure an Zeolithe der Typen DAY oder Mordenit anschließt.

### Beispiel 1 Adsorption von Citronensäure

Es wurden folgende Zeolithe eingesetzt (H-Form, Porengröße ∼ 0,8 nm)

| | |
|---|---|
| Wessalith®-DAY | Modul 200 |
| Wessalith®-DAY | Modul 110 |
| Wessalith®-DAY | Modul 56 |
| Wessalith®-DAY | Modul 25 |

Wessalith®-DAY - dealuminierter Y-Zeolith, Fa. Degussa

### Versuchsbedingungen:

- Temperatur:: 25 ± 3 °C
- Wertstoff:: Citronensäure-Lösungen mit Konzentrationen von 0,5 - 30 Gew.-% Citronen-Säure
- pH-Werte:: Es wurde unter den Bedingungen gearbeitet, die sich beim Zusammenführen von Citronensäure + Zeolith ergaben:≈ 1 - 3
- Verhältnis Zeolith (atro): Wertstofflösung:: 1 : 10

### Versuchsbeschreibung:

Zu jeweils 5 g DAY-Zeolithpulver mit den Moduln 200, 110, 56 und 25 wurden 50 g Citronensäurelösung verschiedener Konzentrationen gegeben und die Suspensionen in einem Erlenmeyerkolben bei Raumtemperatur geschüttelt. Die Referenzproben der Citronensäure-Lösungen enthielten kein Zeolithpulver - wurden aber unter denselben Bedingungen wie die Probelösungen geschüttelt. Die sich nach der Analyse von Referenz- und Gleichgewichtslösungen ergebenden maximalen Gleichgewichtsbeladungen zeigt folgende Tabelle:

| Zeolithtyp: | Maximale Gleichgewichtsbeladung in g Citronens./100 g DAY(atro) |
|---|---|
| Wessalith DAY Modul 200 | 12 |
| Wessalith DAY Modul 110 | 8 |
| Wessalith DAY Modul 56 | 8 |
| Wessalith DAY Modul 25 | 6 |

Das Adsorptionsgleichgewicht war nach maximal 2 Stunden erreicht.

### Beispiel 2: Adsorption und Desorption von Citronensäure

Es wurde ein Zeolith des Typs Wessalith-DAY Modul 200 in Form von 2 mm Vollzylindern eingesetzt (DE-OS 42 02 671 und DE-OS 41 17 202).

### Versuchsbedingungen:

- Temperatur:: 25 ± 3° C
- Wertstoff:: 12 Gew.-% Citronensäurelösung
- Verhältnis Zeolith:: Adsorptionslösung: 1 : 10
- Verhältnis Zeolith:: Desorptionslösung: 1 : 10
- Desorptionslösung:: NH₃-Lösung mit 1,0 g NH₃/lH2O
- pH-Wert Adsorption:: 1 - 2
- pH-Wert Desorption:: ≈ 11 (Startwert) ⇒ ≈ 5 (Endwert)
- Durchsatz Wertstofflösung:: 900 ml/h

### Versuchsbeschreibung:

In eine Festbettsäule wurden ca. 40 g Zeolith-Formkörper vom Typ DAY Modul 200 eingefüllt. Über diese Säule wurde anschließend ≈ 400 ml Citronensäurelösung (12 %ige Lösung) 2 Stunden im Kreislauf gepumpt. Die Gleichgewichtslösung wurde analysiert. Nach dieser Adsorption wurden die Formkörper mit VE-H2O gewaschen, d. h über die Säule wurde 15 Minuten VE-H2O im Kreislauf gepumpt. Das Waschwasser wurde ebenfalls analysiert (VE: vollentsalzt).

Nach dem Waschvorgang wurde die Desorptionslösung (≈ 400 ml) 2 Stunden über die Säule im Kreislauf gepumpt. Die Desorptionslösung wurde im Anschluß auf Citronensäuregehalt analysiert.

Die Adsorptions-Desorptionszyklen wurden mit diesem Zeolith 10 Mal wiederholt.

Die Adsorptionsleistung des Zeolithen bleibt über 10 Zyklen konstant und die Desorption ist jeweils quantitativ.

Das Röntgendiffraktogramm des Zeolithen zeigte nach den 10 Zyklen die Kristallinität des Ausgangsmaterials.

### Beispiel 3: Adsorption von Milchsäure

Es wurden folgende Zeolithe eingesetzt (H-Form)

| | |
|---|---|
| Wessalith-DAY | Modul 200 |
| Wessalith-DAY | Modul 110 |
| Wessalith-DAY | Modul 56 |
| Wessalith-DAY | Modul 25 |
| HZSM5 | Modul 400 |
| H-ZSM5 | Modul 42 |
| H-ZSM5 | Modul 28 |
| H-Mordenit | Modul 30 |

### Versuchsbedingungen:

- Temperatur:: 25 ± 3 °C
- Wertstoff:: Milchsäurelösungen mit Konzentrationen von 1 - 25 Gew.-% Milchsäure
- pH-Werte:: Es wurde unter den Bedingungen gearbeitet, die sich beim Zusammenführen von Milchsäure + Zeolith ergibt pH: 1,5 - 3
- Verhältnis Zeolith(atro) :: Wertstofflösung: 1 : 10

### Versuchsbeschreibung:

Zu jeweils 2,5 g Zeolithpulver wurden 25 g Milchsäurelösung verschiedener Konzentrationen gegeben und die Suspension in einem Erlenmeyerkolben bei Raumtemperatur geschüttelt. Die Referenzprobe der Milchsäurelösung enthielt kein Zeolithpulver. Die maximal ermittelten Gleichgewichtsbeladungen zeigt die folgende Tabelle

| Zeolithtyp: | Maximale Gleichgewichtsbeladung in g Milchs./100 g Zeolith(atro) |
|---|---|
| DAY Modul 200 | 15 |
| DAY Modul 110 | 12 |
| DAY Modul 56 | 12 |
| DAY Modul 25 | 11 |
| H-ZSM-5 Modul 400 | 14 |
| H-ZSM-5 Modul 42 | 10 |
| H-ZSM-5 Modul 28 | 7 |
| H-Mordenit Modul 30 | 5 |

Die maximalen Gleichgewichtsbeladungen bei der Milchsäureadsorption haben sich nach maximal 1 Stunde eingestellt.

### Beispiel 4: Abtrennung von Citronensäure aus Fermentationsbrühen

Es zeigte sich, daß die Abtrennung von Citronensäure (CIT) aus auf unterschiedlicher Basis beruhenden Fermentationsbrühen die bevorzugte Verwendung verschiedener Zeolithtypen erfordert

**Tab. 1**

| | Zeolith Modul | Beladung CIT(%) | Beladung(%) Nebenprod. | Reinheit auf dem Zeolith(%) |
|---|---|---|---|---|
| Melasse | DAY 200 | 7,3 | 1,95 | 78 |
| basis | Mordenit 30 | 4,0 | 0 | 100 |
| Glucose | DAY 200 | 8,7 | 0,15 | 98 |
| basis | Mordenit 30 | 1,1 | 0,5 | 69 |

### Beispiel 5: Adsorption von Nebenprodukten an ZSM-5 aus einer Fermentationsbrühe

Es wurde folgender Zeolith eingesetzt (H-Form)

| | | |
|---|---|---|
| ZSM-5 | Modul 28 | 2 mm VZ |

### Versuchsbedingungen:

- Temperatur:: 25 ± 3 °C
- Werkstoff:: Fermentationsbrühe (FB) mit 5 % Citronensäure, 0,5 % Oxalsäure, 0,5 % Fumarsäure
- pH-Werte:: Es wurde unter den Bedingungen gearbeitet, die sich beim Zusammenführen von FB und Zeolith ergaben: ≈ 1 - 3
- Verhältnis Zeolith (atro):: Wertstofflösung 1 : 3

### Versuchsbeschreibung:

In 40 g ZSM-5 Zeolith-Formkörper (FK) wurden 120 g FB gegeben und in einem Erlenmeyer-Kolben bei Raumtemperatur 20 h geschüttelt. Die Referenzprobe der FB enthielt keine FK, wurde aber unter denselben Bedingungen wie die Probelösung geschüttelt.
Die untersuchten Nebenprodukte werden von ZSM-5 mit abnehmendem Modul zunehmend besser adsorbiert. Mit ZSM-5 Modul 28 konnte die Fumarsäure vollständig und die Oxalsäure zu 70 % aus der Adsorptionslösung entfernt werden.

## Patentansprüche

1. Verfahren zur Abtrennung von Hydroximono- und tricarbonsäuren aus wäßrigen Lösungen durch Adsorption,
**dadurch gekennzeichnet**,
daß man eine mindestens eine dieser Carbonsäuren enthaltende wäßrige Lösung unter Adsorptionsbedingungen mit einem adsorbierenden Material enthaltend einen Zeolith mit einem Modul bis 400 in Kontakt bringt und die entsprechende Carbonsäure daraus durch Desorption gewinnt.

2. Verfahren gemäß Anspruch 1,
**dadurch gekennzeichnet**,
daß die wäßrige Lösung Citronensäure enthält.

3. Verfahren gemäß Anspruch 1,
**dadurch gekennzeichnet**,
daß die wäßrige Lösung Milchsäure enthält.

4. Verfahren gemäß einem oder mehreren der Ansprüche 1 bis 3,
**dadurch gekennzeichnet**,
daß der pH-Wert der Carbonsäurelösung während der Adsorption zwischen 0,5 und 5 liegt.

5. Verfahren gemäß Anspruch 4,
**dadurch gekennzeichnet**,
daß der pH-Wert zwischen 1,0 und 4,0 liegt.

6. Verfahren gemäß Anspruch 2,
**dadurch gekennzeichnet**,
daß man einen dealuminierten Y-Zeolith mit einem Modul zwischen 20 und 400 einsetzt.

7. Verfahren gemäß Anspruch 2,
**dadurch gekennzeichnet**,
daß man einen Zeolith des Typs Mordenit mit einem Modul zwischen 15 und 400 einsetzt.

8. Verfahren gemäß Anspruch 3,
**dadurch gekennzeichnet**,
daß man einen dealuminierten Y-Zeolith mit einem Modul zwischen 20 und 400 oder der Typen ZSM-5 oder Mordenit mit einem Modul zwischen 15 und 400 einsetzt.

9. Verfahren gemäß einem oder mehreren der Ansprüche 1 bis 8,
**dadurch gekennzeichnet**,
daß man die Carbonsäure mit einer wäßrigen Lösung bis zu einem End-pH > 5 desorbiert, insbesondere mit Wasser.

10. Verfahren gemäß Anspruch 9
**dadurch gekennzeichnet**,
daß man mit einer Alkalihydroxid-haltigen Lösung desorbiert.

11. Verfahren gemäß Anspruch 9,
**dadurch gekennzeichnet**,
daß man mit einer ammoniakalischen Lösung, bevorzugt bei einem Anfangs-pH von ∼ 11 desorbiert.

12. Verfahren gemäß einem oder mehreren der Ansprüche 1 bis 8
**dadurch gekennzeichnet**,
daß man als Ausgangslösungen die bei der Herstellung der Säuren anfallenden Fermentationsbrühen verwendet.

13. Verfahren gemäß den Ansprüchen 6 und 12,
**dadurch gekennzeichnet**,
daß man eine Fermentationsbrühe auf Glucosebasis einsetzt.

14. Verfahren gemäß den Ansprüchen 7 und 12,
**dadurch gekennzeichnet**,
daß man eine Fermentationsbrühe auf Melassebasis einsetzt.

15. Verfahren gemäß den Ansprüchen 6, 7 und 12 bis 14,
**dadurch gekennzeichnet**,
daß man die Nebenprodukte Essigsäure, Oxalsäure und Fumarsäure vor der Antrennung der Hydroxycarbonsäuren an einem Zeolith des Typs ZSM-5 adsorbiert.

## Claims

1. A process for removing hydroxymonocarboxylic acids and tricarboxylic acids from aqueous solutions by adsorption, characterised in that an aqueous solution containing at least one of these carboxylic acids is placed in contact, under adsorbing conditions, with an adsorbing material containing a zeolite with a modulus of up to 400 and the corresponding carboxylic acid is recovered therefrom by desorption.

2. A process according to Claim 1, characterised in that the aqueous solution contains citric acid.

3. A process according to Claim 1, characterised in that the aqueous solution contains lactic acid.

4. A process according to one or more or Claims 1 to 3, characterised in that the pH of the carboxylic acid solution is between 0.5 and 5 during adsorption.

5. A process according to Claim 4, characterised in that the pH is between 1.0 and 4.0.

6. A process according to Claim 2, characterised in that a dealuminised Y zeolite with a modulus between 20 and 400 is used.

7. A process according to Claim 2, characterised in that a zeolite of the mordenite type with a modulus between 15 and 400 is used.

8. A process according to Claim 3, characterised in that a dealuminised Y zeolite with a modulus between 20 and 400 or of the ZSM-5 or mordenite type with a modulus between 15 and 400 is used.

9. A process according to one or more of Claims 1 to 8, characterised in that the carboxylic acid is desorbed with an aqueous solution up to a final pH of > 5, in particular using water.

10. A process according to Claim 9, characterised in that an alkali metal hydroxide solution is used for desorbing.

11. A process according to Claim 9, characterised in that an ammoniacal solution, preferably with an initial pH of about 11 is used for desorbing.

12. A process according to one or more of Claims 1 to 8, characterised in that fermentation liquors produced during production of the acids are used as starting solutions.

13. A process according to Claims 6 and 12, characterised in that a fermentation liquor based on glucose is used.

14. A process according to Claims 7 and 12, characterised in that a fermentation liquor based on molasses is used.

15. A process according to Claims 6, 7 and 12 to 14, characterised in that the by-products acetic acid, oxalic acid and fumaric acid are adsorbed on a zeolite of the ZSM-5 type before removing the hydroxycarboxylic acids.

## Revendications

1. Procédé pour la séparation par adsorption d'acides hydroxymonocarboxyliques et hydroxytricarboxyliques de solutions aqueuses, caractérisé en ce qu'on met en contact une solution aqueuse contenant au moins un de ces acides carboxyliques, sous des conditions d'adsorption, avec un matériau adsorbant contenant une zéolithe qui présente un module allant jusqu'à 400 et en ce qu'on obtient l'acide carboxylique correspondant à partir de celui-ci par désorption.

2. Procédé selon la revendication 1, caractérisé en ce que la solution aqueuse contient de l'acide citrique.

3. Procédé selon la revendication 1, caractérisé en ce que la solution aqueuse contient de l'acide lactique.

4. Procédé selon une quelconque des revendications 1 à 3, caractérisé en ce que le pH de la solution contenant l'acide carboxylique est compris entre 0,5 et 5.

5. Procédé selon la revendication 4, caractérisé en ce que le pH est compris entre 1,0 et 4,0.

6. Procédé selon la revendication 2, caractérisé en ce qu'on utilise une zéolithe Y désaluminisée présentant un module compris entre 20 et 400.

7. Procédé selon la revendication 2, caractérisé en ce qu'on utilise une zéolithe de type mordénite présentant un module compris entre 15 et 400.

8. Procédé selon la revendication 3, caractérisé en ce qu'on utilise une zéolithe Y désaluminisée présentant un module compris entre 20 et 400 ou du type ZSM-5 ou du type mordénite présentant un module compris entre 15 et 400.

9. Procédé selon une quelconque des revendications 1 à 8, caractérisé en ce qu'on désorbe l'acide carboxylique avec une solution aqueuse jusqu'à un pH final > 5, en particulier avec de l'eau.

10. Procédé selon la revendication 9, caractérisé en ce qu'on désorbe avec une solution contenant un hydroxyde alcalin.

11. Procédé selon la revendication 9, caractérisé en ce qu'on désorbe avec une solution ammoniacale, de préférence à un pH de départ de ≈ 11.

12. Procédé selon une quelconque des revendications 1 à 8, caractérisé en ce qu'on utilise comme solutions de départ les bouillons de fermentation produits lors de la fabrication des acides.

13. Procédé selon les revendications 6 et 12, caractérisé en ce qu'on utilise un bouillon de fermentation à base de glucose.

14. Procédé selon les revendications 7 et 12, caractérisé en ce qu'on utilise un bouillon de fermentation à base de mélasse.

15. Procédé selon les revendications 6, 7 et 12 à 14, caractérisé en ce qu'on adsorbe les produits secondaires que sont l'acide acétique, l'acide oxalique et l'acide fumarique avant la séparation des acides hydroxycarboxyliques sur une zéolithe de type ZSM-5.
